# EUROPEAN PATENT APPLICATION

(11) **EP 3 162 350 A1**
(43) Date of publication of application: **03.05.2017**
(21) Application number: 15815282.7
(22) Date of filing: 29.06.2015
(51) Int. Cl.: A61J 1/00, A61J 1/10, A61L 31/00

(54) **PHARMACEUTICAL PRODUCT CONTAINER AND PHARMACEUTICAL PREPARATION**

(30) Priority: 30.06.2014 JP 2014135138
(71) Applicant: Taisei Kako Co., Ltd., Kita-ku Osaka-shi Osaka 531-0072 (JP)
(72) Inventor: YANO, Yukihiro, Ibaraki-shi Osaka 567-0054 (JP); FUJISHIMA, Yutaka, Ibaraki-shi Osaka 567-0054 (JP); NAKAMURA, Takayuki, Ibaraki-shi Osaka 567-0054 (JP); SONOYAMA, Tomoyuki, Ibaraki-shi Osaka 567-0054 (JP)
(74) Representative: Stolmár & Partner Patentanwälte PartG mbB
(86) International application number: PCT/JP2015/068612
(87) International publication number: WO 2016/002686

(57) **Abstract**

The present invention relates to a pharmaceutical container formed by a resin composition containing a cyclic olefin resin and a dibenzoylmethane compound. Further, the present invention relates to a pharmaceutical formulation including the aforementioned pharmaceutical container, wherein the pharmaceutical container is internally filled with drug.

## Description

### CROSS-REFERENCE TO RELATED APPLICATION

This application claims priority to Japanese Patent Application No. 2014-135138, the disclosure of which is incorporated herein by reference in its entirety.

### FIELD

The present invention relates to a pharmaceutical container and a pharmaceutical formulation.

### BACKGROUND

Conventionally, a prefilled syringe formulation in which a syringe is filled with drug in advance has been known. As a material used for the syringe of such a prefilled syringe formulation of this type, polypropylene resins and cyclic olefin resins are used, for example. Among these, cyclic olefin resins are suitably used in view of transparency, chemical resistance, and the like (for example, Patent Literature 1).

By the way, there are some drugs whose qualities and properties change due to ultraviolet light. In the case where such a drug is used as the drug of the prefilled syringe formulation, it is necessary to prevent the drug filled in the syringe from being exposed to ultraviolet light.

In order to solve such a problem, a method of covering the syringe filled with the drug by a light-blocking label and a method of housing the prefilled syringe in a packaging medium having light-blocking properties have been proposed (for example, Patent Literatures 2 and 3).

### CITATION LIST

### Patent Literature

Patent Literature 1: JP H10-152566 A
Patent Literature 2: JP 5245189 B
Patent Literature 3: JP 2012-157703 A

### SUMMARY

### Technical Problem

However, in the methods proposed so far, there is a possibility that the light-blocking label is stripped, or the drug in the syringe is exposed to ultraviolet light when the prefilled syringe formulation is taken out from the packaging medium having light-blocking properties. Such a problem occurs not only in the syringe of the prefilled syringe formulation but also in general pharmaceutical containers. Therefore, studies have been made to impart ultraviolet-blocking properties to pharmaceutical containers themselves, and there has been a demand for a pharmaceutical container having excellent transparency and ultraviolet-blocking properties.

The present invention has been devised in view of the problem described above, and an object thereof is to provide a pharmaceutical container having excellent transparency and ultraviolet-blocking properties, and to provide a pharmaceutical formulation including this pharmaceutical container.

### Solution to Problem

The present invention has been accomplished to solve the aforementioned problem and provides a pharmaceutical container formed by a resin composition containing a cyclic olefin resin and a dibenzoylmethane compound.

In the pharmaceutical container, the dibenzoylmethane compound may be at least one selected from 2-methyl dibenzoylmethane, 4-methyl dibenzoylmethane, 4-isopropyl dibenzoylmethane, 4-tert-butyl dibenzoylmethane, 2,4-dimethyl dibenzoylmethane, 2,5-dimethyl dibenzoylmethane, 4,4'-diisopropyl dibenzoylmethane, 4,4'-dimethoxydibenzoylmethane, 4-tert-butyl-4'-methoxydibenzoylmethane, 2-methyl-5-isopropyl-4'-methoxydibenzoylmethane, 2-methyl-5-tert-butyl-4'-methoxydibenzoylmethane, 2,4-dimethyl-4'-methoxydibenzoylmethane, and 2,6-dimethyl-4-tert-butyl-4'-methoxydibenzoylmethane.

In the pharmaceutical container, the dibenzoylmethane compound may be 4-tert-butyl-4'-methoxydibenzoylmethane.

The pharmaceutical container may have a content of the dibenzoylmethane compound in the resin composition of 0.01 wt% or more and 5.0 wt% or less.

The pharmaceutical container may be a syringe, a vial, an ampoule, or an infusion bag.

Further, the present invention provides a pharmaceutical formulation including the aforementioned pharmaceutical container, wherein the pharmaceutical container is internally filled with drug.

### BRIEF DESCRIPTION OF DRAWINGS

Fig. 1A shows a front view of a prefilled syringe formulation according to an embodiment of the present invention.
Fig. 1B shows a sectional view taken along the line A-A in Fig. 1A.
Fig. 2 is a graph showing optical transmittance of resin plates A1 to A4 in a wavelength region of 220 to 780 nm.
Fig. 3 is a graph showing optical transmittance of resin plates B1 to B7 in a wavelength region of 220 to 440 nm.
Fig. 4 is a graph showing optical transmittance of resin plates B8 to B14 in a wavelength region of 220 to 440 nm.

### DESCRIPTION OF EMBODIMENTS

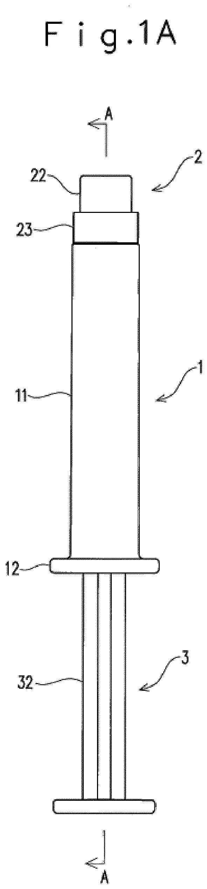

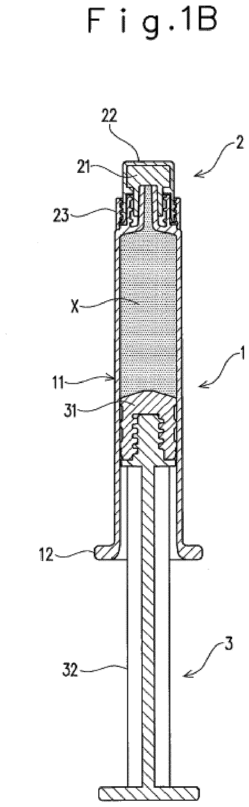

Hereinafter, an embodiment of a pharmaceutical container and a pharmaceutical formulation according to the present invention will be described with reference to Fig. 1A and Fig. 1B.

The pharmaceutical formulation according to this embodiment is a prefilled syringe formulation including a syringe as a pharmaceutical container. Specifically, as shown in Fig. 1A and Fig. 1B, the prefilled syringe formulation includes a cylindrical syringe 1 internally filled with drug X, a cap 2 mounted on the distal end of the syringe 1, and a plunger 3 that is inserted into the syringe 1 and is slidable in the axial direction of the syringe 1. The prefilled syringe formulation is configured so that, when the plunger 3 is pressed, the plunger 3 slides to move within the syringe 1 toward the distal end of the syringe 1, and the drug filled in the syringe 1 is extruded from the distal end of the syringe 1.

The syringe 1 includes a cylindrical syringe body 11 having a nozzle at the distal end, and a finger hold 12 that is arranged at the proximal end of the syringe body 11 in the form of a flange so as to hold a finger. The drug X is filled in the syringe body 11. The syringe 1 of this embodiment is formed by a resin composition containing a cyclic olefin resin and a dibenzoylmethane compound. This allows the syringe 1 of this embodiment to have excellent transparency and ultraviolet-blocking properties.

The thickness of the syringe body 11 in the thinnest portion (sidewall) is generally 0.5 mm to 3.0 mm. Further, the volume of the syringe body 11 is generally 0.5 mL to 2000 mL.

The cap 2 includes a sealing part 21 configured to seal the nozzle provided at the distal end of the syringe body 11, and a cylindrical cap body 22 having the sealing part 21 arranged therein and configured to fix the sealing part 21. Further, the cap 2 includes a cylindrical connecting part 23 mounting the cap body 22 on its inner circumferential portion. The sealing part 21 is formed, for example, by a resin such as rubber. The cap body 22 and the connecting part 23 are formed, for example, by a plastic resin.

The plunger 3 includes a gasket 31 inserted into the syringe body 11 in order to seal the syringe body 11 filled with the drug X, and a plunger rod 32 having a distal end that is threadedly coupled to the gasket 31. The gasket 31 is formed, for example, by an elastic body such as rubber and thermoplastic elastomer. The plunger rod 32 is formed, for example, by a plastic resin.

Examples of the drug X include liquid formulations, granular formulations composed of powder, solid formulations, and dust formulations. The liquid formulations are not limited to aqueous solutions, and they include suspensions, oily solutions, and the like.

Next, the resin composition constituting the syringe 1 of this embodiment will be described in detail.

### (Cyclic olefin resin)

The resin composition constituting the syringe 1 of this embodiment contains a cyclic olefin resin as its main component. This can improve the transparency and chemical resistance of the syringe 1.

Examples of the cyclic olefin resin include resins that are generally used as materials for pharmaceutical containers such as cyclic olefin polymers (COP) and cyclic olefin copolymers (COC). Examples of the COP include a COP containing a repeating unit represented by formula (1) below, and a COP containing a repeating unit represented by formula (2) below. Examples of the commercially available COP include "ZEONEX" manufactured by Zeon Corporation and "ZEONOR" manufactured by Zeon Corporation. Examples of the COC include a COC containing a repeating unit represented by formula (3) below, a COC containing a repeating unit represented by formula (4) below, and a COC containing a repeating unit represented by formula (5) below. Examples of the commercially available COC include "APEL" manufactured by Mitsui Chemicals, Inc. and "TOPAS" manufactured by POLYPLASTICS CO., LTD. One of these can be used alone, or two or more of them can be used in combination. 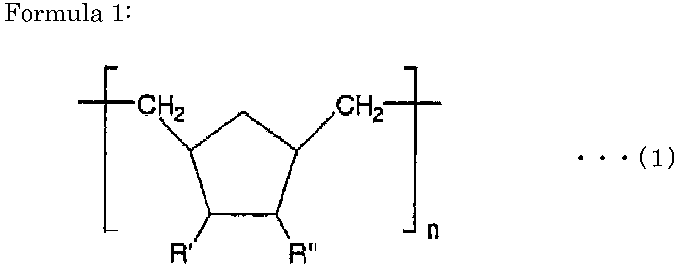 where R' and R" each denote a hydrocarbon group, and n is an integer of 1 or more. 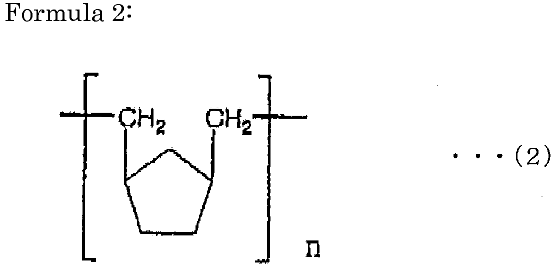 where n is an integer of 1 or more. 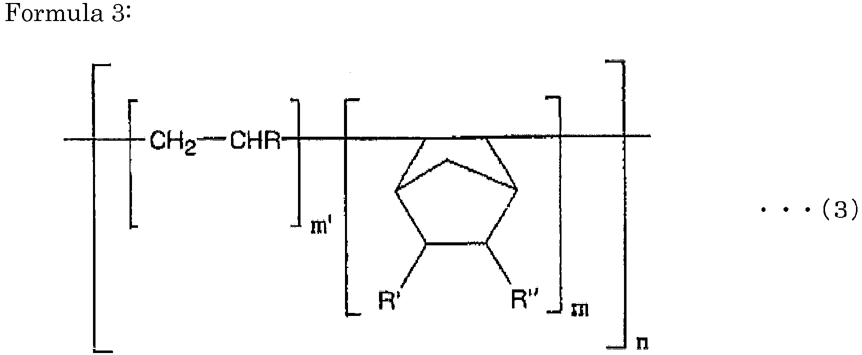 where R, R', and R" each denote a hydrocarbon group, and m, m', and n are each an integer of 1 or more. 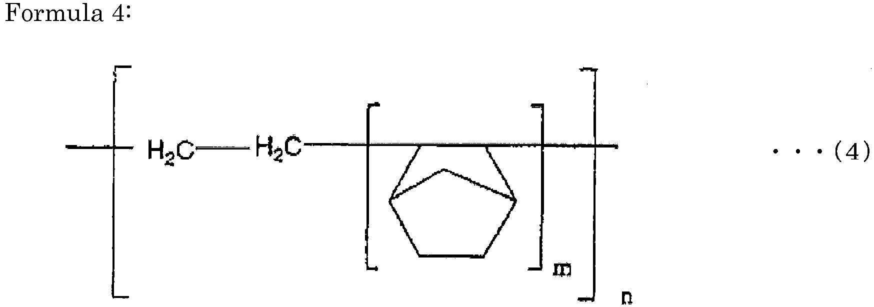 where m and n are each an integer of 1 or more. 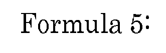 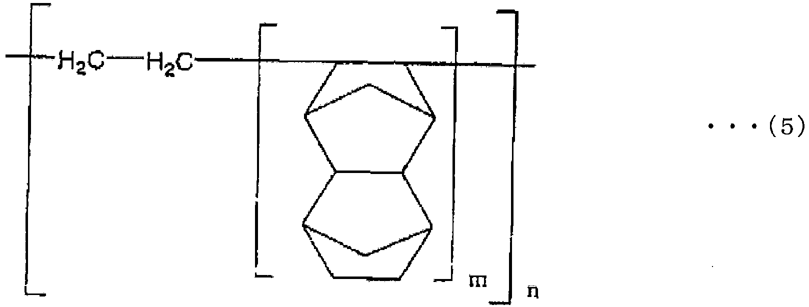 where m and n are each an integer of 1 or more.

The content of the cyclic olefin resin in the resin composition constituting the syringe 1 is preferably 90 wt% or more, more preferably 95 wt% or more, further preferably 99 wt% or more, in view of the transparency, chemical resistance, moisture barrier properties, and the like. Further, the content is preferably 99.99 wt% or less, more preferably 99.98 wt% or less, further preferably 99.95 wt% or less.

The glass transition temperature (Tg) of the cyclic olefin resin is preferably 115°C or more, more preferably 121°C or more, further preferably 129°C or more, in order to reduce the possibility of deformation due to high-pressure steam sterilization. Further, in view of the formability, the temperature is preferably 300°C or less, more preferably 250°C or less, further preferably 200°C or less. In this description, the glass transition temperature (Tg) can be determined based on the method described in Japanese Industrial Standards (JIS) K7121:1987 "Method for measuring transition temperature of plastics".

### (Dibenzoylmethane compound)

The resin composition constituting the syringe 1 of this embodiment contains a dibenzoylmethane compound. By containing the dibenzoylmethane compound that is an ultraviolet absorber, the resin composition can give ultraviolet-blocking properties to the syringe 1 without reducing the transparency of the syringe 1 and can suppress the transmission of ultraviolet light (wavelength region: 220 to 380 nm) into the syringe 1.

Examples of the dibenzoylmethane compound include 2-methyl dibenzoylmethane, 4-methyl dibenzoylmethane, 4-isopropyl dibenzoylmethane, 4-tert-butyl dibenzoylmethane, 2,4-dimethyl dibenzoylmethane, 2,5-dimethyl dibenzoylmethane, 4,4'-diisopropyl dibenzoylmethane, 4,4'-dimethoxydibenzoylmethane, 4-tert-butyl-4'-methoxydibenzoylmethane, 2-methyl-5-isopropyl-4'-methoxydibenzoylmethane, 2-methyl-5-tert-butyl-4'-methoxydibenzoylmethane, 2,4-dimethyl-4'-methoxydibenzoylmethane, and 2,6-dimethyl-4-tert-butyl-4'-methoxydibenzoylmethane. Among these, 4-tert-butyl-4'-methoxydibenzoylmethane is preferable.

The content of the dibenzoylmethane compound in the resin composition constituting the syringe 1 is preferably 0.01 wt% or more, more preferably 0.05 wt% or more, in view of the ultraviolet-blocking properties. Further, in view of the transparency and dissolution properties, the content is preferably 5.0 wt% or less, more preferably 0.6 wt% or less.

### (Other components)

The resin composition constituting the syringe 1 of this embodiment may further contain other components such as a stabilizer, a pigment, a dye, an antiblocking agent, an antistatic agent, and an antioxidant, without significantly impairing the effects of this embodiment.

### (Forming method of syringe)

The syringe 1 of this embodiment is produced, for example, by mixing the aforementioned components constituting the resin composition and forming the mixture using a general forming method such as injection molding, press molding, and extrusion molding.

### (Method for producing prefilled syringe formulation)

The prefilled syringe formulation of this embodiment is obtained, for example, by attaching the cap 2 to the distal end of the syringe 1 in a sterile environment after sterilization of the syringe 1 of this embodiment, filling the syringe 1 with sterilized drug, and attaching the plunger 3 in order to seal the syringe 1 filled with the drug. The sterilization may be performed after filling the syringe 1 of this embodiment with the drug. In this case, the filling of the syringe 1 with the drug and the attachment of the cap 2 and the plunger 3 are not necessarily performed in a sterile environment.

The pharmaceutical container according to the present invention is formed by a resin composition containing a cyclic olefin resin and a dibenzoylmethane compound.

Further, the pharmaceutical formulation according to the present invention includes the aforementioned pharmaceutical container, and the pharmaceutical container is internally filled with drug.

The present invention can provide a pharmaceutical container having excellent transparency and ultraviolet-blocking properties, and a pharmaceutical formulation including the pharmaceutical container.

The pharmaceutical container and the pharmaceutical formulation according to the present invention are not limited to the aforementioned embodiment, and various modifications can be made without departing from the gist of the present invention.

In the aforementioned embodiment, the syringe of the prefilled syringe formulation is employed as a pharmaceutical container, but there is no limitation to this. Any container that directly contacts with the drug like injection containers such as a syringe of a general injector, a vial, an ampoule, and an infusion bag can be applied to the pharmaceutical container. Further, in the aforementioned embodiment, the prefilled syringe formulation is employed as a pharmaceutical formulation, but there is no limitation to this. Any formulation which includes the aforementioned pharmaceutical container and in which the pharmaceutical container is internally filled with drug can be applied to the pharmaceutical formulation.

In the aforementioned embodiment, an injection needle may be attached to the distal end of the syringe.

### EXAMPLES

Next, the present invention will be described further in detail by way of examples. However, the present invention is not limited to these examples.

### [Test A]

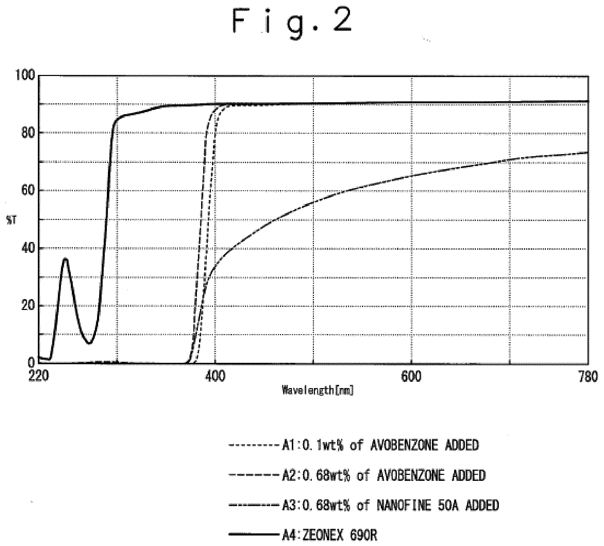

In test A, the relationship between the concentration of the ultraviolet absorber and the optical transmittance was evaluated as follows. First, resin plates A1 to A4 (10 mm × 40 mm × 1 mm) constituted by a resin composition composed of components shown below were prepared. Then, the total light transmittance was measured using an ultraviolet/visible spectrophotometer ("V-650" manufactured by JASCO Corporation). Fig. 2 shows the measurement results. The content of each component added as the ultraviolet absorber is expressed as a content of the component in the entire resin composition (the same applies to the following tests).

Resin plate A1: Cyclic olefin resin (COP: product name "ZEONEX 690R" manufactured by Zeon Corporation) + Ultraviolet absorber (4-tert-butyl-4'-methoxydibenzoylmethane (hereinafter referred to also as avobenzone), Content: 0.1 wt%)
Resin plate A2: Cyclic olefin resin (COP: product name "ZEONEX 690R" manufactured by Zeon Corporation) + Ultraviolet absorber (Avobenzone, Content: 0.68 wt%)
Resin plate A3: Cyclic olefin resin (COP: product name "ZEONEX 690R" manufactured by Zeon Corporation) + Ultraviolet absorber (Zinc oxide: product name "NANOFINE 50A" manufactured by Sakai Chemical Industry Co., Ltd., Content: 0.68 wt%)
Resin plate A4: Cyclic olefin resin (COP: product name "ZEONEX 690R" manufactured by Zeon Corporation)

From the results shown in Fig. 2, it was confirmed that light in the wavelength region of 220 to 380 nm (ultraviolet light) can be substantially blocked in the resin plates A1 to A3 to which the ultraviolet absorber was added. Further, the resin plate A1 with a small content of avobenzone showed substantially the same ultraviolet-blocking properties as the resin plate A2 with a large content of avobenzone. That is, it turned out that avobenzone even if it is added in a small amount has excellent ultraviolet absorptivity. It was confirmed that the resin plates A1 and A2 to which avobenzone was added as the ultraviolet absorber have high transmittance of light (visible light) in the wavelength region (380 to 780 nm), as compared with the resin plate A3 to which zinc oxide was added as the ultraviolet absorber, and it turned out that they have excellent transparency.

### [Test B]

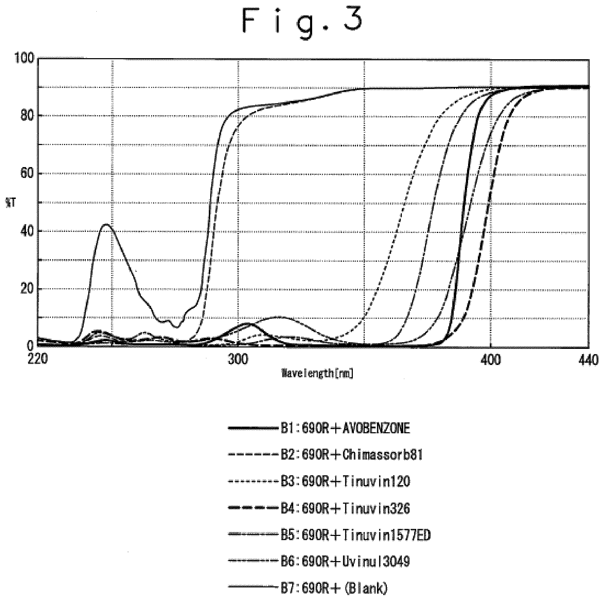

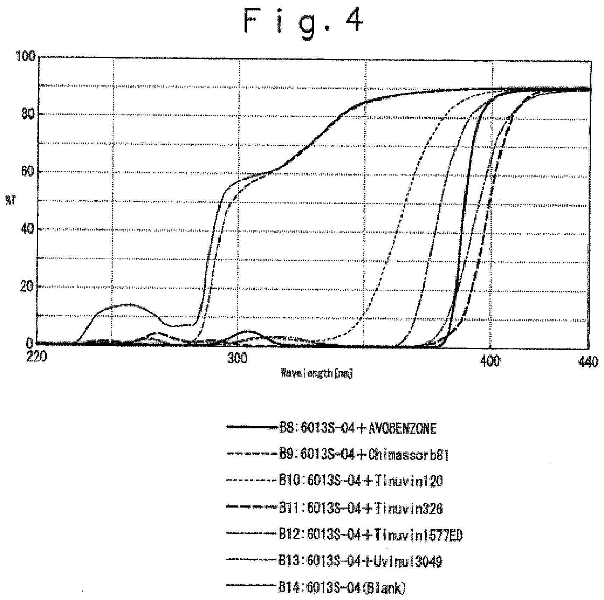

In test B, the ultraviolet blocking effect by various ultraviolet absorbers was studied as follows. First, resin plates B1 to B14 (thickness: 1 mm) constituted by a resin composition composed of components shown below were prepared. Then, the center portion of each of the resin plates B1 to B14 was cut out into a size of 10 mm × 40 mm. Then, the total light transmittance was measured using an ultraviolet/visible spectrophotometer ("V-650" manufactured by JASCO Corporation). Fig. 3 shows the optical transmittance in a wavelength of 220 to 440 nm of the resin plates B1 to B7. Further, Fig. 4 shows the optical transmittance in a wavelength of 220 to 440 nm of the resin plates B8 to B14. Then, in Fig. 3 and Fig. 4, the peak area in a wavelength of 220 to 380 nm of the resin plates B1 to B6 and B8 to B13 was determined, and Table 1 shows the results.

Resin plate B1: Cyclic olefin resin (COP: product name "ZEONEX 690R" manufactured by Zeon Corporation) + Ultraviolet absorber (Avobenzone, Content: 0.1 wt%)
Resin plate B2: Cyclic olefin resin (COP: product name "ZEONEX 690R" manufactured by Zeon Corporation) + Benzophenone ultraviolet absorber ("Chimassorb81" manufactured by BASF SE, Content: 0.1 wt%)
Resin plate B3: Cyclic olefin resin (COP: product name "ZEONEX 690R" manufactured by Zeon Corporation) + Benzoate ultraviolet absorber ("Tinuvin120" manufactured by BASF SE, Content: 0.1 wt%)
Resin plate B4: Cyclic olefin resin (COP: product name "ZEONEX 690R" manufactured by Zeon Corporation) + Benzotriazole ultraviolet absorber ("Tinuvin326" manufactured by BASF SE, Content: 0.1 wt%)
Resin plate B5: Cyclic olefin resin (COP: product name "ZEONEX 690R" manufactured by Zeon Corporation) + Triazine ultraviolet absorber ("Tinuvin1577ED" manufactured by BASF SE, Content: 0.1 wt%)
Resin plate B6: Cyclic olefin resin (COP: product name "ZEONEX 690R" manufactured by Zeon Corporation) + Benzophenone ultraviolet absorber ("Uvinul3049" manufactured by BASF SE, Content: 0.1 wt%)
Resin plate B7: Cyclic olefin resin (COP: product name "ZEONEX 690R" manufactured by Zeon Corporation)
Resin plate B8: Cyclic olefin resin (COC: product name "TOPAS6013S-04" manufactured by POLYPLASTICS CO., LTD.) + Ultraviolet absorber (Avobenzone, Content: 0.1 wt%)
Resin plate B9: Cyclic olefin resin (COC: product name "TOPAS6013S-04" manufactured by POLYPLASTICS CO., LTD.) + Benzophenone ultraviolet absorber ("Chimassorb81" manufactured by BASF SE, Content: 0.1 wt%) Resin plate B10: Cyclic olefin resin (COC: product name "TOPAS6013S-04" manufactured by POLYPLASTICS CO., LTD.) + Benzoate ultraviolet absorber ("Tinuvin120" manufactured by BASF SE, Content: 0.1 wt%)
Resin plate B11: Cyclic olefin resin (COC: product name "TOPAS6013S-04" manufactured by POLYPLASTICS CO., LTD.) + Benzotriazole ultraviolet absorber ("Tinuvin326" manufactured by BASF SE, Content: 0.1 wt%) Resin plate B12: Cyclic olefin resin (COC: product name "TOPAS6013S-04" manufactured by POLYPLASTICS CO., LTD.) + Triazine ultraviolet absorber ("Tinuvin1577ED" manufactured by BASF SE, Content: 0.1 wt%)
Resin plate B13: Cyclic olefin resin (COC: product name "TOPAS6013S-04" manufactured by POLYPLASTICS CO., LTD.) + Benzophenone ultraviolet absorber ("Uvinu13049" manufactured by BASF SE, Content: 0.1 wt%) Resin plate B14: Cyclic olefin resin (COC: product name "TOPAS6013S-04" manufactured by POLYPLASTICS CO., LTD.)

**Table 1**

| RESIN PLATE | PEAK AREA |
|---|---|
| B1 | 184.722 |
| B2 | 1586.19 |
| B3 | 7777.16 |
| B4 | 208.233 |
| B5 | 564.709 |
| B6 | 537.224 |
| B8 | 121.132 |
| B9 | 1658.46 |
| B10 | 6600.43 |
| B11 | 195.535 |
| B12 | 352.597 |
| B13 | 206.508 |

From the results shown in Table 1, the peak area in a wavelength of 220 to 380 nm was small in the resin plates B1 and B8 to which avobenzone was added, as compared with the resin plates B2 to B6 and B9 to B13 to which other ultraviolet absorbers were added. It can be seen from this that avobenzone has excellent ultraviolet absorptivity as compared with the other ultraviolet absorbers. Further, as shown in Fig. 3 and Fig. 4, it was confirmed that avobenzone has a transmittance gradient around a wavelength of 380 to 400 nm that is nearly perpendicular, as compared with the other ultraviolet absorbers. Further, it was confirmed that avobenzone hardly absorbs light in the visible light region at 400 nm or more, as compared with the other ultraviolet absorbers. That is, it turned out that avobenzone hardly reduces the transparency of the resin plates.

From the above, it turned out that ultraviolet-blocking properties can be given to the pharmaceutical container syringe without reducing the transparency of the pharmaceutical container when avobenzone is added to the resin composition constituting the pharmaceutical container as an ultraviolet absorber.

### [Elution test]

Pharmaceutical containers, particularly, injection containers are required to satisfy "Requirements of plastic containers for aqueous injections" of Japanese Pharmacopoeia so as not to affect the safety and stability of the drug filled in the container. Therefore, the elution test shown below was performed, and whether or not "Requirements of plastic containers for aqueous injections" are satisfied was evaluated.

### <Preparation of samples>

First, resin plates C1 to C4 (80.0 mm × 50.0 mm × 1.0 mm) each constituted by a resin composition composed of components shown below were prepared, and a portion of each resin plate having a thickness as uniform as possible was cut out. The thickness was about 1 mm.

Resin plate C1: Cyclic olefin resin (COP: product name "ZEONEX 690R" manufactured by Zeon Corporation) + Ultraviolet absorber (Avobenzone, Content: 0.1 wt%)
Resin plate C2: Cyclic olefin resin (COP: product name "ZEONEX 690R" manufactured by Zeon Corporation) + Ultraviolet absorber (Zinc oxide: product name "NANOFINE 50A" manufactured by Sakai Chemical Industry Co., Ltd., Content: 0.68 wt%)
Resin plate C3: Cyclic olefin resin (COP: product name "ZEONEX 690R" manufactured by Zeon Corporation)
Resin plate C4: Cyclic olefin resin (COP: product name "ZEONEX 690R" manufactured by Zeon Corporation) + Ultraviolet absorber (Avobenzone, Content: 0.6 wt%)

Next, cut pieces of each of the resin plates C1 to C4 were collected so that the total of front and back surface areas was about 600 cm², and these pieces were further chopped to a size with a length of about 5 cm and a width of 0.5 cm, followed by washing with water and subsequent drying at room temperature, so that each piece served as a test piece. Then, each test piece was put into a flask (volume: about 300 mL), to which 200 mL of purified water was accurately added, and the flask was sealed. Then, it was heated at 121°C for 1 hour using a high-pressure steam sterilizer and thereafter was allowed to stand to room temperature. This content fluid was used as a test solution.

Further, the same operation was applied to water, so that a blank test solution was prepared. Then, the following tests were performed on each test solution and the blank test solution.

### <Foaming test>

5 mL of the test solution was put into a stoppered test tube with an inner diameter of about 15 mm and a length of about 200 mm, which was vigorously mixed by shaking for 3 minutes. The time until generated bubbles almost disappear was measured. When the measured time is within 3 minutes, it was determined to be suitable. Table 2 shows the results of the foaming test.

**Table 2**

| RESIN PLATE | ULTRAVIOLET ABSORBER | TIME FOR BUBBLE DISAPPEARANCE (Sec) | DETERMINATION |
|---|---|---|---|
| C1 | AVOBENZONE (0.1 wt%) | 0 | SUITABLE |
| C2 | ZINC OXIDE (0.68 wt%) | 0 | SUITABLE |
| C3 | - | 0 | SUITABLE |
| C4 | AVOBENZONE (0.6 wt%) | 0 | SUITABLE |

### <pH test>

20 mL of each of the test solution and the blank test solution was extracted, to which 1.0 mL of potassium chloride with a concentration of 0.1% was added. Then, the pH of each of the test solution and the blank test solution was measured using a pH meter, and their difference was calculated. When the calculated difference is 1.5 or less, it was determined to be suitable. Table 3 shows the results of the pH test.

**Table 3**

| RESIN PLATE | ULTRAVIOLET ABSORBER | pH | | DIFFERENCE (mL) | DETERMINATION |
|---|---|---|---|---|---|
| | | TEST SOLUTION | BLANK TEST SOLUTION | | |
| C1 | AVOBENZONE (0.1 wt%) | 5.43 | 5.37 | 0.06 | SUITABLE |
| C2 | ZINC OXIDE (0.68 wt%) | 5.54 | 5.37 | 0.17 | SUITABLE |
| C3 | - | 5.40 | 5.37 | 0.03 | SUITABLE |
| C4 | AVOBENZONE (0.6 wt%) | 5.48 | 5.37 | 0.11 | SUITABLE |

### <Test for detecting potassium permanganate reducing material>

20 mL the test solution was put into a stoppered Erlenmeyer flask, to which 20.0 mL of a potassium permanganate solution at 0.002 moL/L and 1 mL of dilute sulfuric acid were added, and thereafter the flask was sealed and boiled for 3 minutes. After cooling the flask with water, 0.10 g of a potassium iodide solution was added thereto, the flask was sealed and mixed well, and then was allowed to stand still for 10 minutes. Thereafter, 5 drops of starch reagent as an indicator were added thereto, and the mixture was titrated with a sodium thiosulfate solution at 0.01 moL/L. Further, the same operation was applied to 20 mL of the blank test solution as to the test solution. Then, the difference in the consumed amount of potassium permanganate solution at 0.002 moL/L between the test solution and the blank test solution was calculated. When the difference in the consumed amount was 1.0 mL or less, it was determined to be suitable. Table 4 shows the results of the test for detecting potassium permanganate reducing material.

**Table 4**

| RESIN PLATE | ULTRAVIOLET ABSORBER | TITRATED AMOUNT (mL) | | DIFFERENCE (mL) | DETERMINATION |
|---|---|---|---|---|---|
| | | TEST SOLUTION | BLANK TEST SOLUTION | | |
| C1 | AVOBENZONE (0.1 wt%) | 20.0 | 20.0 | 0.0 | SUITABLE |
| C2 | ZINC OXIDE (0.68 wt%) | 19.7 | 20.0 | 0.3 | SUITABLE |
| C3 | - | 20.1 | 20.0 | 0.1 | SUITABLE |
| C4 | AVOBENZONE (0.6 wt%) | 19.9 | 20.0 | 0.1 | SUITABLE |

### <Test for detecting ultraviolet absorption spectrum absorbing material>

The absorbance of the test solution was measured with a spectrophotometer using the blank test solution as a control. Here, measurement was performed at four fixed wavelengths of 220 nm, 240 nm, 241 nm, and 350 nm. Then, when the absorbance at a wavelength of 220 nm or more and less than 241 nm is 0.08 abs or less, and the absorbance at a wavelength of 241 nm or more and 350 nm or less is 0.05 abs or less, it was determined to be suitable. Table 5 shows the results of the test for detecting ultraviolet absorption spectrum absorbing material.

**Table 5**

| RESIN PLATE | ULTRAVIOLET ABSORBER | ABSORBANCE (abs) | | DETERMINATION |
|---|---|---|---|---|
| | | 220 to 240 nm | 241 to 350 nm | |
| C1 | AVOBENZONE (0.1 wt%) | 0.005 | 0.004 | SUITABLE |
| C2 | ZINC OXIDE (0.68 wt%) | 0.016 | 0.003 | SUITABLE |
| C3 | - | 0.011 | 0.004 | SUITABLE |
| C4 | AVOBENZONE (0.6 wt%) | 0.013 | 0.012 | SUITABLE |

### <Test for detecting evaporation residue>

After sensing the heat of an evaporating dish and slowly cooling it to room temperature, the empty weight was measured. Then, 10 mL of the test solution was put on the evaporating dish and was evaporated to dryness on a water bath. Then, the residue was dried for 1 hour in a thermostatic bath set to 105°C. Thereafter, the evaporating dish was taken out and was slowly cooled to room temperature, and the weight was measured. The weight of the evaporation residue was determined from the difference between the measured weight and the empty weight. When the weight of the evaporation residue in 10 mL of the test solution is 1.0 mg or less, it was determined to be suitable. Table 6 shows the results of the test for detecting evaporation residue.

**Table 6**

| RESIN PLATE | ULTRAVIOLET ABSORBER | WEIGHT OF WEIGHING BOTTLE (mg) | WEIGHT OF WEIGHING BOTTLE AFTER DRYING (mg) | WEIGHT OF EVAPORATION RESIDUE (mg) | DETERMINATION |
|---|---|---|---|---|---|
| C1 | AVOBENZONE (0.1 wt%) | 46033.50 | 46033.75 | 0.25 | SUITABLE |
| C2 | ZINC OXIDE (0.68 wt%) | 45970.60 | 45970.64 | 0.03 | SUITABLE |
| C3 | - | 47533.44 | 47533.59 | 0.15 | SUITABLE |
| C4 | AVOBENZONE (0.6 wt%) | 45679.37 | 45679.31 | 0.00 | SUITABLE |

As shown in Tables 2 to 6, it was confirmed that all the resin plates C1 to C4 showed suitable determination results in the aforementioned tests. Further, the elution test was conducted also on the resin plates B2 to B6 used in the aforementioned test B, in the same manner as for the aforementioned resin plates C1 to C4, and it was confirmed that all showed suitable results.

From these results, it turned out that it is possible not only to impart ultraviolet-blocking properties to a pharmaceutical container without reducing the transparency of the pharmaceutical container, but also to ensure the safety of the pharmaceutical container by employing avobenzone as an ultraviolet absorber in the resin composition constituting the pharmaceutical container.

### REFERENCE SIGNS LIST

- 1:: Syringe
- 11:: Syringe body
- 12:: Finger hold
- 2:: Cap
- 21:: Sealing part
- 22:: Cap body
- 23:: Connecting part
- 3:: Plunger
- 31:: Gasket
- 32:: Plunger rod
- X:: Drug

## Claims

1. A pharmaceutical container formed by a resin composition comprising:
a cyclic olefin resin; and
a dibenzoylmethane compound.

2. The pharmaceutical container according to claim 1, wherein
the dibenzoylmethane compound is at least one selected from 2-methyl dibenzoylmethane, 4-methyl dibenzoylmethane, 4-isopropyl dibenzoylmethane, 4-tert-butyl dibenzoylmethane, 2,4-dimethyl dibenzoylmethane, 2,5-dimethyl dibenzoylmethane, 4,4'-diisopropyl dibenzoylmethane, 4,4'-dimethoxydibenzoylmethane, 4-tert-butyl-4'-methoxydibenzoylmethane, 2-methyl-5-isopropyl-4'-methoxydibenzoylmethane, 2-methyl-5-tert-butyl-4'-methoxydibenzoylmethane, 2,4-dimethyl-4'-methoxydibenzoylmethane, and 2,6-dimethyl-4-tert-butyl-4'-methoxydibenzoylmethane.

3. The pharmaceutical container according to claim 2, wherein
the dibenzoylmethane compound is 4-tert-butyl-4'-methoxydibenzoylmethane.

4. The pharmaceutical container according to any one of claims 1 to 3, having a content of the dibenzoylmethane compound in the resin composition of 0.01 wt% or more and 5.0 wt% or less.

5. The pharmaceutical container according to any one of claims 1 to 4, being a syringe, a vial, an ampoule, or an infusion bag.

6. A pharmaceutical formulation comprising:
the pharmaceutical container according to any one of claims 1 to 5, wherein
the pharmaceutical container is internally filled with drug.
